# EUROPÄISCHE PATENTSCHRIFT

(11) **EP 0 775 530 B1**
(45) Veröffentlichungstag und Bekanntmachung des Hinweises auf die Patenterteilung: **26.02.2003**
(21) Anmeldenummer: 96116728.5
(22) Anmeldetag: 18.10.1996
(51) Int. Cl.: B05B 11/00

(54) **Spender mit zwei Fördereinheiten**
Dispenser comprising two dispensing units
Distributeur avec deux unitées à débiter

(30) Priorität: 08.11.1995 DE 19541594
(43) Veröffentlichungstag der Anmeldung: 28.05.1997
(73) Patentinhaber: ING. ERICH PFEIFFER GMBH, 78315 Radolfzell (DE)
(72) Erfinder: Ritsche, Stefan, D-78315 Radolfzell (DE)
(74) Vertreter: Patentanwälte Ruff, Wilhelm, Beier, Dauster & Partner

(56) Entgegenhaltungen:
- EP-A- 0 318 834
- EP-A- 0 427 609
- FR-A- 2 641 337
- US-A- 4 773 562
- US-A- 4 826 048
- US-A- 4 902 281
- US-A- 5 169 029
- US-A- 5 224 627
- US-A- 5 301 841
- US-A- 5 411 176

## Beschreibung

Die Erfindung betrifft eine Austrag-Einheit für Medien, die fließfähig, beispielsweise gasförmig, flüssig, breiig bzw. gelartig und/oder pulverförmig sein können, und zwar jeweils vor oder nach dem Austrag aus der Einheit.

Die Austag-Einheit kann jeweils für sich oder in baulicher Kombination ein- oder mehrfach einen Grundkörper, einen Austrag-Förderer, eine Austrag-Betätigung, einen AuslaßKanal, Austrag-Öffnungen und eine in einem Grundkörper eingesetzte Baueinheit enthalten, die ein Betätigungsstößel, ein Auslaßventil oder ein anderer Innenkörper oder -Bereich sein kann, welcher einen Austrag- bzw. Auslaßkanal im Querschnitt oder dergleichen begrenzen kann.

Die Austrag-Einheit kann nur einen von zwei Grundkörpern bzw. Betätigungs-Einheiten enthalten, welche zur Betätigung des Austrages gegeneinander drehend und/oder axial gegeneinander mit einer einzigen Hand manuell zu bewegen sind, so daß diese Betätigungs-Einheit dann für den Gebrauch der Austrag-Einheit mit der weiteren Betätigungs-Einheit, z.B. durch Aufstecken zu verbinden ist. Zweckmäßig sind jedoch beide Betätigungs-Einheiten vormontiert und gegeneinander beweglich zusammengesetzt, so daß zur Betätigung des Austrages nur noch ihre gegenseitige Bewegung erforderlich ist. Eine der Betätigungs-Einheiten enthält die Austragöffnungen und die andere Betätigungs-Einheit den Medienspeicher, wobei die beiden Betätigungs-Einheiten bzw. Grundkörper auch einteilig ausgebildet sein könnten.

Zweckmäßig weist der eine Grundkörper zwei bzw. mehr Austrag-Öffnungen auf, von denen mindestens eine zur Ablösung des Mediums von der Austrag-Einheit ins Freie mündet, während eine oder mehrere weitere Austrag-Öffnungen innerhalb des zugehörigen Grundkörpers in Strömungsrichtung vor der erstgenannten Austrag-Öffnung gleichgerichtet, achsgleich oder voneinander abweichend gerichtet liegen können. Sollen zwei Medien gesondert ausgebracht werden, so liegen zwei ins Freie führende Austrag-Öffnungen nebeneinander in wenigstens einer der zuletztgenannten Anordnungen, z.B. so, daß die beiden Medien unmittelbar im Anschluß an den Austritt miteinander vermischen und so ein chemisch und/oder physikalisch reagierendes Mischmedium bilden. Hat z.B. das Mischmedium aufgrund der Reaktion eine weniger gute Fließfähigkeit als eines oder beide der Ausgangsmedien, z.B. weil diese bei der Vermischung durch Molekül-Vernetzung in einen gelartigen Zustand übergehen, so ist es zweckmäßig, diese beiden Medien bis kurz vor, während oder unmittelbar nach dem Austrag ins Freie vollständig gesondert zu halten und z.B. erst im Abstand von der Austragfläche der Austrag-Einheit zusammenzuführen. Eine gute Durchmischung ergibt sich dabei insbesondere, wenn mindestens eines der Medien in möglichst feiner Verteilung dem anderen zugeführt wird.

Aus der US 5 411 176 ist dabei ein Austragförderer für Medien bekannt, der einen Grundkörper mit einer darin eingesetzten Baueinheit aufweist. Die Austragseinheit verfügt dabei über einen Austragsförderer sowie eine Austragsbetätigung an mehreren Betätigungsstellen und wenigstens einem Auslasskanal.

Der Erfindung liegt die Aufgabe zugrunde, eine Austrag-Einheit so zu gestalten, daß Aufbau und Montage der Einheit vereinfacht ist.

Erfindungsgemäß kann eine innenliegende und/oder eine ins Freie führende Austrag-Öffnung durch einen Ausgang einer Zerstäuberdüse gebildet sein, aus welcher das zugehörige Medium feinstverteilt austritt. Werden gleichzeitig zwei Austrag-Öffnungen von gesondert zugeführten Medien durchströmt, so können diese dadurch sehr gut vermischt werden. Dies wird noch weiter verbessert, wenn zwei oder mehr solche Austrag-Öffnungen jeweils durch einen gesonderten Ausgang, gesonderter Zerstäuberdüsen gebildet sind und z.B. die Zerstäubungskegel dieser Düsen erst in geringem Abstand von den Austrag-Öffnungen mit großen bzw. größten Teilen ihrer Querschnitte ineinander greifen.

Jede Zerstäuberdüse weist zweckmäßig eine Düsen-Austrag-öffnung von weniger als einem oder einem halben Millimeter Innenweite, eine dieser unmittelbar vorgelagerte Einrichtung zur Verwirbelung des zugehörigen Mediums bzw. zur Drallerzeugung um die Düsenachse und/oder einen in dieser Weise unmittelbar vorgelagerten Innenkörper bzw. Düsenkern auf, welcher durch einen von derjenigen Wand gesonderten Bauteil gebildet ist, die von der Düsenöffnung durchsetzt ist. Dieser Innenkörper kann auch einen zugehörigen Kanalabschnitt des zur Austrag-Öffnung führenden Auslaßkanales begrenzen, und zwar im Quer- und/oder Längsschnitt durch den Kanalabschnitt bzw. den Innenkörper alleine und/oder gemeinsam mit einem weiteren Bauteil, z.B. demjenigen, welcher von der Austrag-Öffnung durchsetzt ist.

Unabhängig von der beschriebenen Ausbildung sind zweckmäßig zwei, gesonderten Austrag-Öffnungen bzw. Auslaßkanälen zugehörige Innenkörper zu einer insbesondere einteiligen Baueinheit zusammengefaßt, welche vorgefertigt an dem zugehörigen Grundkörper befestigt bzw. gelagert werden kann. Entsprechend können auch zwei Anschlüsse für zwei gesonderte Austragförderer durch eine solche bzw. diese Baueinheit gebildet sein. Bei einer Austrag-Einheit mit nur einer einzigen, in's Freie führenden Austrag-Öffnung bzw. mit nur einem einzigen Austragförderer oder Medienspeicher, kann diese Baueinheit aber auch nur einen einzigen Innenkörper und einen einzigen Anschluß enthalten. Zweckmäßig weist die Baueinheit am Ende des Innenkörpers bzw. des Anschlusses einen Tragteil zur Befestigung am Grundkörper auf, so daß die Baueinheit mit dem Grundkörper vormontiert und dann mit dem Austragförderer oder dergleichen verbunden werden kann. Die Befestigung am Grundkörper erfolgt vorteilhaft durch eine Haftverbindung, wie Schweißen, oder über eine selbsteinrastende bzw. federnde Schnappverbindung, deren Schnappglieder in der gleichen Richtung miteinander zu verbinden sind, in welcher auch der Innenkörper einzusetzen bzw. der Anschluß mit dem Austragförderer zu verbinden ist. Der Tragteil kann einen oder mehrere Längs- und/oder Querabschnitte des Auslaßkanales begrenzen, welcher zweckmäßig von der Auslaßöffnung des Austragförderers ventilfrei bis zur Austrag-Öffnung durchgeht.

Die Austrag-öffnungen können in einer gemeinsamen Axialebene eine in Axialansicht flach ovale, elliptische oder am äußersten Umfang ähnlich langgestreckt abgerundete Wand durchsetzen, deren größte Erstreckung parallel zur Verbindungsgeraden der Austrag-Öffnungen liegt. Die Begrenzung dieser Wand bildet somit einander gegenüberliegende Bereiche stärker Krümmung und quer dazu einander gegenüberliegende Bereiche geringerer Krümmung. Entgegen Strömungsrichtung geht der an die Begrenzung dieser Wand anschließende Außenumfang des zugehörigen Bauteiles, z.B. eines Austragstutzens, in eine Form über, in der die Krümmungsunterschiede zwischen den genannten Abschnitten geringer werden. Zum Beispiel kann der Außenumfang dieses Bauteiles an seinem von der Austrag-Öffnung entfernten Ende annähernd kreisrund mit einem Durchmesser sein, welcher der größeren Erstreckung der Stirnwand entspricht, so daß die Form der Stirnwand durch in Achsrichtung stetige einseitige oder gegenüberliegende Abflachung dieses kreisrunden oder ähnlichen Umfanges erzielt ist. Eine solche Ausbildung ist auch bei Vorhandensein nur einer einzigen, ins Freie führenden bzw. letzten Austrag-Öffnung für viele Anwendungsfälle vorteilhaft.

Außer für reversierend arbeitende Austrag-Vorrichtungen, die z.B. mindestens eine Pumpe bzw. mindestens einen mit einem Druck-Treibmittel geladenen und durch das Öffnen eines Ventiles zu betätigenden Medienspeicher und meist einen über einen Betätigungshub manuell anzutreibenden und von selbst in entgegengesetzter Richtung zur Ausgangsstellung zurückkehrenden Betätigungs- bzw. Austragkopf aufweisen, sind die erfindungsgemäßen Ausbildungen auch für sogenannte Einmal-Austragvorrichtungen oder dergleichen geeignet. Bei diesen schließt der Auslaßkanal unmittelbar an den Medienspeicher an, welcher den Pump- bzw. Druckraum bildet. Im Falle einer Schubkolbenpumpe taucht dabei der Verdränger bzw. Pumpkolben beweglich in ein Ende des Druckraumes ein, an dessen Innenumfang der Kolben abgedichtet und axial verschiebbar mit einer Kolbenlippe gleitet. Das dem Verdränger gegenüberliegende Ende des Druckraumes kann geschlossen sein, wenn nach Entleerung seine Wiederfüllung mit Medium aufgrund eines Rückhubes nicht vorgesehen ist. Nachdem der Verdränger dieses gegenüberliegende Ende erreicht hat, ist der gesamte Medienspeicher im wesentlichen vollständig entleert.

Diese und weitere Merkmale gehen außer aus den Ansprüchen auch aus der Beschreibung und den Zeichnungen hervor, wobei die Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können. Ausführungsbeispiele der Erfindung sind in den Zeichnungen dargestellt und werden im folgenden näher erläutert. In den Zeichnungen zeigen:
- Fig. 1: eine erfindungsgemäße Austrag-Einheit in teilweise geschnittener Ansicht,
- Fig. 2: die Vorrichtung gemäß Fig. 1 in Draufsicht und geringfügig abgewandelter Ausbildung,
- Fig. 3: einen Teilschnitt durch die Vorrichtung nach Fig. 1 in vergrößerter Darstellung,
- Fig. 4: einen Ausschnitt der Fig. 1 in wesentlich vergrößerter Darstellung,
- Fig. 5: einen Schnitt nach den Linien V-V in Fig. 4,
- Fig. 6: einen Schnitt nach den Linien VI-VI in Fig. 4, und
- Fig. 7: einen Schnitt nach den Linien VII-VII in Fig. 4.

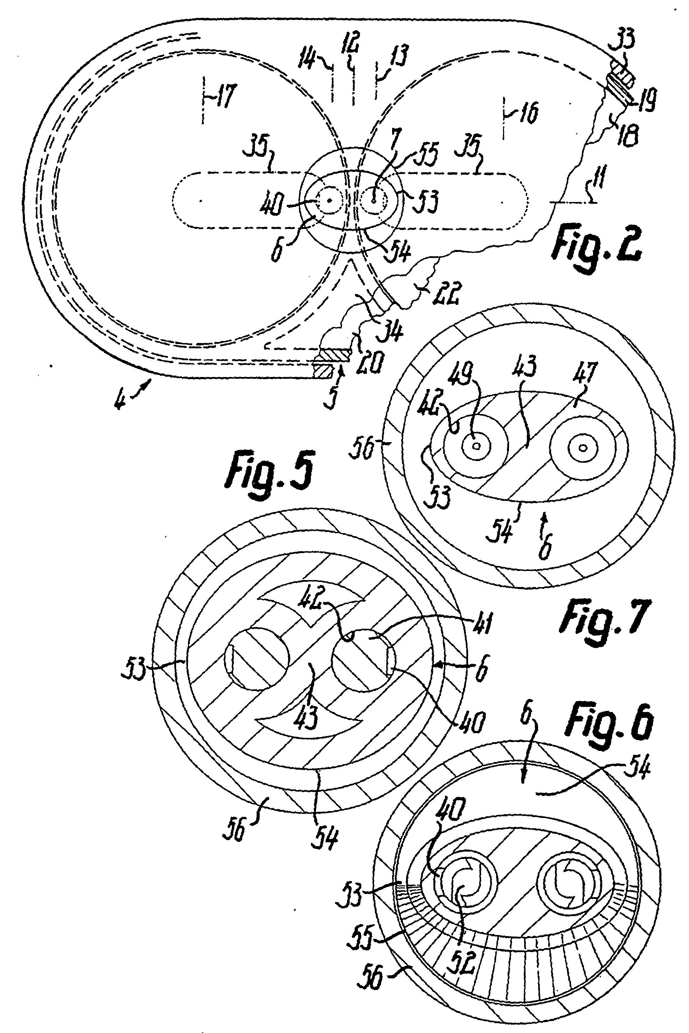

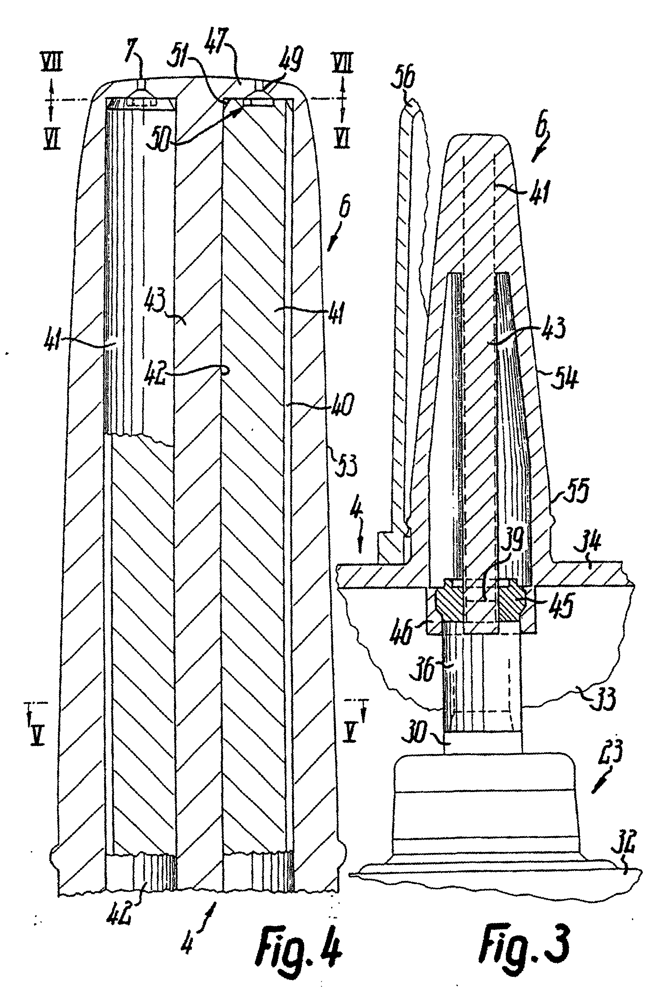

Gemäß der Erfindung ist mindestens eine der Einheiten 1 bis 3 vorgesehen, wobei zum Austrag des Mediums die Betätigungseinheiten 2, 3 zur Austrags-Einheit 1 zusammengesetzt und mit den Fingern nur einer einzigen, die Einheit 1 frei tragenden Hand durch Druck gegeneinander zu bewegen bzw. über einen maximalen Hub von weniger als 10 bzw. 5 mm zu verschieben sind. Jede Einheit 2, 3 weist einen gesonderten, durchgehend einteiligen Grundkörper 4 bzw. 5 aus Kunststoff auf, welche in Axialansicht annähernd deckungsgleiche Form haben. Der längere Grundkörper 4 schließt einen frei über seine Außenseite vorstehenden Austragstutzen 6 mit einer einzigen, zwei oder mehr an seinem freien Ende axial austretenden Austrag-Öffnungen 7 ein. Bei derseits des Stutzens 6 bildet dieser Körper 4 seitlich an den Stutzen 6 anschließende Handhaben 8 bzw. Finger-Druckflächen und eine entsprechende, davon wegweisende Handhabe 9 bildet der Körper 5 mit seinem davon abgekehrten Ende. Die Anordnungen 1 bis 9 liegen im wesentlichen achsparallel oder symmetrisch zu bzw. in einer LängsMittelachse 10, welche quer- bzw. rechtwinklig zueinander liegenden Axial-bzw. Mittelebenen 11, 12 definiert, die auch Symmetrieebenen der Anordnungen 1 bis 9 sind. In Richtung der Mittelebene 11 haben die Anordnungen 1 bis 6, 8 und 9 eine mindestens 1,5- und höchstens 3-fach größere Erstreckung als in Richtung der Ebene 12. Symmetrisch beiderseits der Ebene 12 und parallel zu dieser sind zwei Axialebenen 13, 14 der Austrag-Öffnungen 7 definiert. Jeder Austrag-Öffnung 7 wird Medium durch eine gesonderte FörderEinheit 15 gesondert zugeführt und die zur Achse 10 parallelen Achsen bzw. die zu den Ebenen 12 bis 14 parallelen Axialebenen 16, 17 dieser Einheiten 15 liegen symmetrisch beiderseits der Ebene 12 mit einem gegenüber dem Abstand der Ebenen 13, 14 mehrfach größeren Abstand, so daß sie gemäß Fig. 2 weit außerhalb des Stutzens 6 vorgesehen sind.

Die Körper 4, 5 bilden gemeinsam einen nach außen geschlossenen, in Axialansicht flachovalen Gehäuseraum, in welchem die Einheiten 15 vollständig ummantelt und verdeckt angeordnet sind. Die Einheiten 15 sind form- und/oder kraftschlüssig lagestarr am Körper 5 gehaltert, welche hierfür gesonderte, gehäuseförmige Aufnahmen 15 bildet. Der Körper 5 weist einen radial äußersten Mantel 19 etwa gleicher Länge wie seine größte Weite auf, welcher an einem Ende mit einer Stirnwand 20 verschlossen und am anderen Ende zum Einsetzen bzw. linearen Einstecken der Einheiten 15 offen ist. Der Mantel 19 ist durch eine symmetrisch zur Ebene 12 liegende Trennwand 21 in die beiden, zylindrischen Aufnahmetaschen 18 unterteilt. Diese sind an flaschenförmige Medien-Speicher 22 der Einheiten 15 derart zylindrisch angepaßt, daß jeweils ein Speicher 22 in einer Aufnahme 18 dann durch Umfangsklemmung bzw. federnde Schnapphalterung gegen axiales Herausziehen lagegesichert ist, wenn sein Boden an der Innenseite der Stirnwand 20 durch Anschlag anliegt, wobei der Speicher 22 vollständig innerhalb der Aufnahme 15 liegt. Aus einer durch einen verengten Halsabschnitt gebildeten Speicheröffnung steht ein Austragförderer 23, wie eine Schubkolbenpumpe aus den Anordnungen 5, 22 frei vor. Der Förderer 23 ist zur Bildung der Einheit 15 als vormontierte Fördereinheit in den Speicher 22 achsgleich eingesetzt, dessen erweiterter Bauch einteilig mit dem Hals aus Glas oder dergleichen ausgebildet ist. Der Förderer 23 weist ein langgestrecktes Fördergehäuse 24 auf, das aus einem in den Speicherbauch hineinragenden Zylindergehäuse und einem außerhalb des Speichers 22 liegenden Gehäusedeckel zusammengesetzt ist. Innerhalb des Zylindergehäuses und des Halses des Speichers 22 ist ein Pump- bzw. Druckraum 25 begrenzt, welcher mit einer axial verschiebbaren Kolbeneinheit 26 manuell volumenveränderbar ist.

Durch einen stutzenförmigen Einlaß 27 wird über ein druckabhängig arbeitendes Einlaßventil 28 Medium aus dem Speicher 22 in den Druckraum 25 beim Rückhub der Einheit 26 angesaugt. Beim gegen die Handhabe 9 gerichteten Pumphub wird dieses Medium über ein druck- und/oder wegabhängig öffnendes bzw. schließendes Auslaßventil 29 durch einen Austragkanal 31 eines Betätigungsstößels 30 hindurch aus dem Druckraum 25 herausgepreßt. Der Betätigungsstößel 30 ist durch das den Gehäusedeckel verschiebbar durchsetzende Ende des Kolbenstößels der Einheit 26 gebildet. Das Gehäuse 24 ist mit einer Befestigung 32, insbesondere einem außenliegenden Krimpring lagestarr und axialverspannt so an einem Außenbund des Speicherhalses befestigt, daß die Speicheröffnung mit einer das Gehäuse 24 umgebenden Dichtung verschlossen ist, die zwischen die Stirnflächen von Außenbunden des Halses und des Gehäuses 24 eingreift und innerhalb der die Außenbunde umgreifenden Befestigung 32 liegt.

Der den Körper 5 kappenförmig als Deckel an der offen Seite schließende Grundkörper 4 enthält einen äußersten, entgegengesetzt zum Mantel 19 frei ausragenden Mantel 33, welcher an seinem von Stirnwand 20 abgekehrten Ende in eine Stirnwand 34 übergeht. Die Außenseiten der quer oder rechtwinklig zur Achse 10 bzw. den Ebenen 11 bis 17 liegenden Platten oder Stirnwände 34, 20 bilden die Handhaben 8, 9 und reichen jeweils nur bis zum Außenumfang des zugehörigen Mantels 33 bzw. 19. Der Innenumfang des Mantels 33 gleicht dem Außenumfang des Mantels 19, den er in allen Funktionsstellungen mit geringstem Spaltabstand axial verschiebbar übergreift, obwohl auch der Mantel 19 den Mantel 33 übergreifen könnte. Die vorstehenden Teile der Einheiten 15 ragen in den Mantel 33. Die Bestandteile 23 bis 32 jeder Einheit 15 liegen in der zugehörigen Achse bzw. Ebene 16, 17. Beide Speicher 22 reichen unmittelbar bis an die dünne Trennwand 21, so daß ihr lichter Zwischenabstand der geringsten Dicke dieser Wand 21 entspricht.

Der Grundkörper 4 trägt beweglich, z.B. axialbeweglich, oder lagestarr festsitzend eine einteilige Baueinheit 35, die vollständig verdeckt innerhalb des Körpers 4 bzw. von dessen Kappe und/oder von dessen Stutzen 6 liegt. Der Körper 4 ist mit jeweils einem von der Innenseite seiner Stirnwand 34 frei vorstehenden, hülsenförmigen Anschluß 36 am zugehörigen Stößel 30 form- und/oder kraftschlüssig befestigt, der einteilig mit der formsteifen Einheit 35 aus Kunststoff ausgebildet ist und von dieser frei zur Handhabe 9 vorsteht. Der Stößel 30 greift mit seinem Außenumfang abgedichtet in den Anschluß 36 bzw. dessen Innenumfang ein, welcher mit seiner Endfläche an einer Schulter des Stößel 30 anliegt.

Die in der Endfläche des Stößel 30 liegende Auslaßöffnung jedes Austragkanales 31 ist über einen gesonderten Auslaßkanal 37 ventilfrei mit der zugehörigen Austragöffnung 7 stets leitungsverbunden. Der mehrfach abgewinkelte Auslaßkanal 37 bildet innerhalb des Anschlußes 36 und an den Austragkanal 31 unmittelbar anschließend einen ersten Längsabschnitt 38, welcher in Strömungsrichtung an ein Ende eines Querabschnittes 39 anschließt, der seinerseits in Strömungsrichtung an ein Ende eines gegenüber beiden Abschnitten 38, 39 wesentlich verengten Längsabschnittes 40 anschließt. Der Abschnitt 39 liegt an der Innenseite der Stirnwand 34 und ist im Querschnitt sowohl von dieser als auch von der Einheit 35 über den Umfang vollständig geschlossen begrenzt. Die Einheit 35 ist hierzu an ihrer an der Stirnwand 34 anliegenden Seite mit gesonderten Nuten so versehen, daß der Abschnitt 39 vom Abschnitt 38 bzw. der Ebene 16 und 17 zur Achse 10 bzw. den Ebenen 12 bis 14 quer oder rechtwinklig zu diesen durchgehend geradlinig gerichtet ist. Beide Abschnitte 39 fluchten miteinander. Die zur Achse 10 bzw. den Ebenen 11 bis 17 parallelen Abschnitte 40 durchsetzen den Stutzen 6 an voneinander abgekehrten Seiten der Ebene 12 bis 14 und liegen durchgehend in der Ebene 11. Sie gehen mit konstantem Strömungsquerschnitt vom jeweiligen Abschnitt 39 annähernd bis zur Austragöffnung 7 bzw. bis zu einem zugehörigen Wirbelerzeuger durch und schließen unmittelbar an das zugehörige Ende des jeweiligen Abschnittes 39 an. Die Länge des Stutzens 6 ist mehrfach größer als seine kleinste oder größte Außenweite.

Jeder Kanalabschnitt 40 liegt innerhalb einer gesonderten, bohrungsartigen Aufnahme 42, welche die Stirnwand 34 und den Stutzen 6 annähernd bis zu dessen freiem Ende mit konstanter Weite durchsetzt und einen stabförmigen, beispielsweise zylindrischen Innenkörper 41 eng angepaßt bzw. mit Umfangsklemmung aufnimmt. Die beiden, in den Ebenen 13, 14 liegenden Aufnahmen 42 sind durch eine Trennwand des Körpers 4 bzw. 6 gegeneinander abgedichtet, die vom freien Ende des Stutzen 6 bis zu dessen anderen Ende und über die Innenseite der Stirnwand 34 vorsteht. Die Dicke der Trennwand 43 ist etwa gleich der Wandungs- bzw. Manteldicke der Elemente 4 bis 6 und an ihren voneinander abgekehrten Seiten liegen die Innenkörper 41 so an, daß die Abschnitte 40 an den voneinander abgekehrten Mantel-Seiten der Innenkörper 41 liegen. Jeder Innenkörper 41 steht mit seinem von der Austrag-Öffnung 7 abgekehrten inneren Ende 44 über die Innenseite der Stirnwand 34 vor, bildet die zugehörige Endbegrenzung des Abschnittes 39 und schließt einteilig an die Einheit 35 an.

Die Einheit 35 schließt einen plattenförmigen, zur Achse 10 bzw. den Ebenen 11 bis 17 quer oder rechtwinklig liegenden, parallel zur Ebene 11 langgestreckten Tragteil 45 ein, an den das innere Ende 44 jedes der gradlinigen Innenkörper 41 unmittelbar benachbart zur Trennwand 43 einteilig so anschließt, daß er nur über eine Plattenseite vorsteht. Nur über die davon abgekehrte Plattenseite und radial gegenüber den Innenkörpern 41 nach außen versetzt, stehen die zueinander und zu den Innenkörpern 41 achsparallelen Anschlüsse 36 vor. Der Tragteil 45 begrenzt die Kanalabschnitte 39 und die inneren Enden der Kanalabschnitte 40, wobei sich sein Rand auch über den freien Außenumfang der Anschlüsse 36 als Wulst erstreckt.

Mit seinem ununterbrochen über die Wulste durchgehenden Rand ist nur der Tragteil 45 der Einheit 35 mit einer Befestigung 46 an der Innenseite der Stirnwand 34 gehaltert. Der Körper 4 bildet eine über die Innenseite der Stirnwand 34 vorstehende Hülse oder einen Bund 46, in dessen Innenumfang der Rand der Einheit 35, 45 gegen axiale, radiale und drehende Bewegungen spielfrei gesichert sowie abgedichtet eingreift. Der Innenumfang der Befestigung 46 und der Außenumfang des Tragteiles 45 bzw. der Anschlüsse 36 bilden federnd sowie abdichtend zusammenwirkende Sicherungs- bzw. Schnappglieder, welche nach federnder Aufweitung der Befestigung 46 einrasten, wenn der Bauteil 35 beim Einsetzen der Innenkörper 41 am Ende seiner gegen die Innenseite der Stirnwand 34 gerichteten axialen Montagebewegung an der Innenseite der Stirnwand 34 anschlägt und dadurch die Enden der Abschnitte 38 sowie die zugehörigen offenen Nut-Längsseiten der Abschnitte 39 durch diese Innenseite dicht geschlossen sind. Der Tragteil 45 und die Innenseite der Stirnwand 34 können an ihren einander zugekehrten Seiten entlang des Randes des Tragteiles 45 einen oder mehrere ringförmig ununterbrochen durchgehende Vorsprünge aufweisen, die jeweils dicht in eine entsprechende Nutvertiefung des anderen Bauteiles eingreifen und dadurch eine Labyrintdichtung bilden. Jeder Anschluß 36 bzw. Innenkörper 41 könnte gemäß Fig. 2 einen gesonderten Tragteil aufweisen bzw. durch eine gesonderte Einheit 35 gebildet sein, jedoch ist zweckmäßig für beide Einheiten 15 bzw. beide Austrag-Öffnungen 7 eine gemeinsame, einteilige Einheit 35 vorgesehen.

Jeder Kanalabschnitt 40 liegt über die gesamte Länge des zugehörigen Innenkörpers 41 an dessen Außenumfang, der mit einer entsprechenden Längsnut versehen ist, so daß der Abschnitt 40 durch den Außenumfang des Innenkörpers 41 und den Innenumfang der Aufnahme 42 im Querschnitt über den Umfang geschlossen ist. Die Nut geht bis zur zugehörigen Seite des Tragteiles 45, bis zum inneren Ende des Innenkörpers 41 und bis zur Bodenfläche der Quernut 39 durch. Der Stutzen 6 weist im Radialabstand um einen oder zwei Innenmäntel einen äußersten Stutzenmantel auf, dessen inneres Ende einteilig die Stirnwand 34 anschließt und dessen äußeres Ende einteilig in eine von den Austrag-Öffnungen 7 durchsetzte, zur Mittelachse 10 bzw. den Ebenen 11 bis 14, 16, 17 rechtwinklige Stirnwand 47 übergeht.

Gemeinsam mit den äußeren Enden 51 der Innenkörper 41 bildet diese Stirnwand 47 jeweils eine Zerstäuber-Düse 48, deren Düsenaustritt unmittelbar die jeweilige Austrag-Öffnung 7 bildet, ab welcher das Medium beim Austrag jeweils vollständig von dem Medienspender 1 freigekommen ist. Die Austrag-Öffnung 7 ist durch das äußere Ende eines die Stirnwand 47 durchsetzenden Düsenkanales 49 gebildet, dessen inneres Ende konisch erweitert und über eine Wirbel- bzw. Dralleinrichtung 50 an das Ende des zugehörigen Abschnittes 40 angeschlossen sein kann. Diese Einrichtung 50 liegt zweckmäßig zwischen der Innenfläche der Stirnwand 47 und der dieser zugekehrten Endfläche des zugehörigen Innenkörpers 41, welche gemäß Fig. 6 an ihren Außenumfang anschließende vertiefte Wirbelkanäle 52 aufweisen kann. Der Wirbelkanal 52 der jeweiligen Einrichtung 50 kann im Zentrum um die Düsenachse eine Rotationskammer bilden, an welche zwei oder mehr Radial- bzw. Tangentialkanäle vom Außenumfang des Innenkörpers 41 bzw. vom Innenumfang der Aufnahme 42 her anschließen. Zweckmäßig ist der Außenumfang des Endes 51 mit einer Schrägfläche bzw. Anfasung versehen, an welche die Tangentialkanäle einander quer zur Ebene 11 diametral gegenüberliegend außerhalb des Abschnittes 40 anschließen, so daß entlang dieser Fase ein beiderseits an das Ende des Abschnittes 40 anschließender Ringkanal gebildet ist. Das durch den Abschnitt 40 geförderte Medium strömt aus dessen Ende zunächst in entgegengesetzten Richtungen in den Ringkanal und dann erst von dort in die Tangentialkanäle und schließlich in die Rotationskammern, von wo aus es unmittelbar mit einer Drallströmung in den Düsenkanal 49 und aus der Austrag-Öffnung 7 herausgelangt. Im Ringkanal und in der Einrichtung 50 liegt die Strömungsrichtung des Mediums quer bzw. rechtwinklig zur Strömungsrichtung im Abschnitt 40 bzw. im Düsenkanal 49.

Das äußere Ende des Stutzens 6 bzw. die Stirnwand 47 ist in Axialansicht am Außenumfang parallel zur Ebene 11 langgestreckt flachoval bzw. elliptisch, wobei die Achsen der Austrag-Öffnungen 7 in den Achsen der Kleinkreise dieser Ellipse oder demgegenüber geringfügig zur Ebene 12 versetzt liegen können. Dadurch bildet der Außenumfang des Stutzen 6 über den Umfang zwei einander gegenüberliegende Bereiche 53 stärkerer Krümmung und zwei quer dazu einander gegenüberliegende Bereiche 54 geringerer Krümmung, wobei beide Bereiche 53 einerseits sowie beide Bereiche 54 andererseits gleiche Krümmung haben und jeweils tangential ununterbrochen bzw. glatt ineinander gegenübergehen. Das innere, an die Stirnwand 34 anschließende Ende des Außenumfanges des Stutzens 6 ist demgegenüber kreisrund oder gleichförmig polygonal, wobei dessen Weite geringfügig größer als die größte Weite zwischen den Bereichen 53 bzw. wesentlich größer als die geringste Weite zwischen den Bereichen 54 am äußeren Stutzenende ist. Die Bereiche 53, 54 gehen vom Stutzenende stetig konisch und absatzfrei in den konstant gekrümmten Fußumfang 55 des inneren Stutzenendes so über, daß sich die elliptische Querschnittsform am freien Stutzenende in Richtung zum Stutzenfuß unter gegenseitiger Krümmungsangleichung der Bereiche 53, 54 stetig der Form 55 dieses Fußes annähert. Am freien Endabschnitt des Stutzens 6 ist die Konizität nur der Bereiche 53 etwas stärker als am restlichen Stutzen.

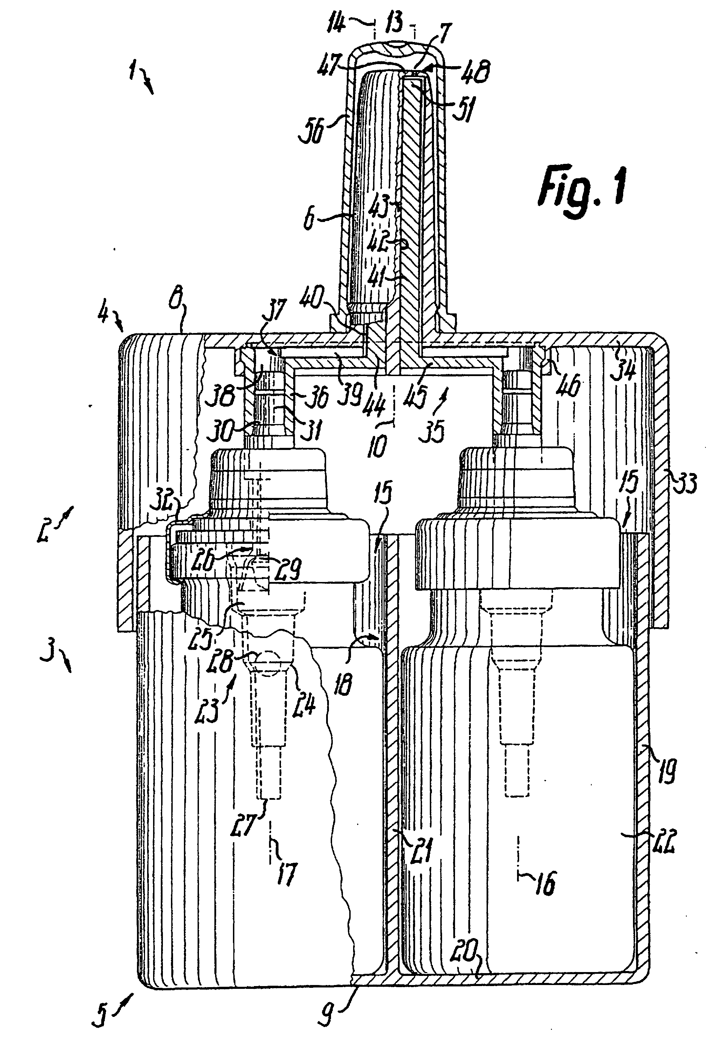

Zum Austrag von Medium wird der Spender 1 in einer Hand so gehalten, daß der Daumen an der Handhabe 9 und zwei weitere Finger an den Handhaben 8 einander gegenüberliegend anliegen. Gegen die Kraft von innerhalb der beiden Gehäuse 24 bzw. Kammern 25 liegenden Rückstellfedern, die auch gleichzeitig Schließfedern der Ventile 28, 29 sein können, werden die beiden Einheiten 2, 3 dann zusammengedrückt, wobei der Körper 5 weiter in den Körper 4 und gegebenenfalls die Anschlüsse 36 in die Gehäuse 24 bis zum Anschlag eintauchen. Der Körper 4 nimmt dabei ausschließlich über die Anschlüsse 36 die beiden Kolbeneinheiten 26 der beiden Fördereinheiten 15 gegenüber deren übrigen Bestandteilen 22, 24, 32 und dem Körper 5 gleichzeitig oder zeit- bzw. wegverzögert mit, so daß beide Druckräume 25 durch die Pumpkolben bzw. Pumpverdränger verengt werden. Die in den Druckräumen 25 enthaltenen Medien werden dadurch unter Druck gesetzt, wodurch die Ventile 28 geschlossen gehalten werden. Nach einem Teil der Pumphubes oder an dessen Ende öffnen die Auslaßventile 29 gleichzeitig oder gegeneinander zeitverzögert, wodurch die Medien aus den Druckräumen 25 durch die Kolbeneinheiten 26 hindurch in die Kanalabschnitte 38 ausgetrieben werden. Von diesen gelangen sie unter Querumlenkung unmittelbar in die Kanalabschnitte 39 und dann nochmals unter entgegengesetzter Umlenkung in die Kanalabschnitte 40, bis sie in der beschriebenen Weise durch die Austrag-Öffnungen 7 als spitz- bzw. stumpfwinklige Sprühkegel austreten. Die beiden Strahle oder Sprühkegel greifen im Abstand außerhalb der Stirnwand 47 ineinander ein und bilden dadurch eine Mischzone, in welcher die feinstverteilten Partikel der beiden, aus den Öffnungen 7 austretenden Medienkomponenten schlagartig gleichmäßig miteinander vermischt werden. Durch entsprechende Wahl der Komponenten führt diese Vermischung der für sich dünnflüssigen Medien zu einer Medienreaktion, z.B. zum Gelieren, d.h., daß das Mischmedium dann wesentlich dickflüssiger ist, z.B. so dickflüssig, daß sein entsprechend feinstverteilter Austrag aus einer Düse entsprechend den Düsen 48 nicht möglich wäre. Das gelierte Mischmedium kann trotzdem seinen feinstverteilten Zerstäubungszustand im wesentlichen beibehalten oder es kann eine Reaktionsverzögerung aufweisen, so daß es erst nach Auftreffen der Sprühstrahle auf eine Fläche, z.B. die Nasenschleimhaut, die genannte Reaktion hat. Nach dem genau dosierten Austritt der Medien werden die Handhaben 8, 9 entlastet, wodurch allein die genannten Rückstellfedern die Einheiten 2, 3 sowie die Förderer 23 gemeinsam wieder in ihre Ausgangslage gemäß Fig. 1 zurückführen, so daß eine gesonderte, unmittelbar an den Bauteilen 4, 5, 35 angreifende Rückstellfeder nicht erforderlich ist. Bei diesem Rückhub wird auch wieder Medium aus den Speichern 22 in die Druckräume 25 angesaugt. Aus der wieder erreichten Ausgangsstellung kann der Spender 1 sofort wieder in der beschriebenen Weise für einen weiteren Austrag betätigt werden.

Bei einem Spender mit nur einer einzigen Austrag-Öffnung, nur einem einzigen Auslaßkanal bzw. nur einer einzigen Fördereinheit 15 könnten diese Anordnungen in der Achse 10 liegen, so daß auch die Bauteile 36, 41 achsgleich angeordnet werden könnten. In diesem Fall, nämlich bei Vorhandensein nur eines einzigen Innenkörpers 41 bzw. einer einzigen Aufnahme 42 wäre die Trennwand 43 nicht erforderlich, die beim dargestellten Ausführungsbeispiel an den Innenumfang der Befestigung 46 einteilig anschließt und bis zu deren freien Ende reicht.

Im Falle eines sogenannten Einmal-Spenders könnte der Innenkörper 48 bzw. die Einheit 35 oder die Hülse bzw. der Anschluß 36 unmittelbar den oder die Pumpverdränger bzw. Kolben als gesonderte Bauteile tragen oder auch einteilig bilden und dieser Kolben würde dann unmittelbar am Innenumfang des oder der Speicher 42 gleiten, so daß dessen Speicherraum gleichzeitig den Druckraum bildet. Der jeweilige Speicher könnte dann einteilig mit dem Körper 5 ausgebildet sein. Ferner könnte über die Innenseite der Stirnwand 34 radial außerhalb des Innenumfangs des Stutzens 6 eine Hülse des Körpers 4 frei vorstehen, welche radial außerhalb des Speicherraumes am Außenumfang von dessen Mantel bzw. eines diesen im Abstand umgebenden Mantels des Körpers 5 geführt ist. Diese Mäntel könnten in der Ausgangsstellung und/oder mindestens einer weiteren Arbeits-Stellung über eine Verriegelung bzw. eine Schnappverbindung ineinandergreifen, welche durch eine gegenseitige Drehbewegung der Einheiten 2, 3 und/oder durch Aufbringen einer genügend und starken Druckkraft an den Handhaben 8, 9 zu Entriegeln ist. So kann z.B. die Vorspannkraft zur Überwindung dieser Verriegelung zu einer schlagartig sehr schnell einsetzenden und starken Pumpbewegung führen. Die Handhabe 8 bzw. die Stirnwand 34 und/oder die Handhabe 9 bzw. die Stirnwand 20 kann auch jeweils über den Außenumfang des zugehörigen Mantels 33 bzw. 19 als Ringbund bzw. streifenförmig vorstehen, so daß insbesondere dann die genannte Verriegelung unmittelbar zwischen den Mänteln 19, 33 vorgesehen sein kann.

Desweiteren kann der Körper 5 einen Außenmantel aufweisen, welcher den Mantel 33 am Außenumfang in mindestens einer oder allen Betriebsstellungen umgibt. Ferner kann der Innenkörper 41 an einer von der Stirnwand 47 mit Radialabstand innerhalb des Außenmantels abstehenden Innenhülse des Körpers 4 befestigt sein bzw. eine beispielsweise hohle Nadel aus Stahl oder dergleichen enthalten oder vollständig durch diese gebildet sein. Der Speicher bzw. Druckraum wäre dann mit dem Kolben dicht verschlossen und in Ausgangsstellung würde die Spitze der Nadel einem Solldurchbruch dieses Kolbens unmittelbar gegenüberliegen. Am Anfang des Pumphubes würde dann die Nadel den Kolben durchstoßen und so den innerhalb und/oder außerhalb ihr liegenden Auslaßkanal erst dann an den Druckraum anschließen, wonach im weiteren Verlauf des Hubes der Kolben durch Anschlag an eine Schulter des Innenkörpers mitgenommen und das Medium ausgetrieben wird.

Das Auslaßventil könnte auch durch ein Schlauchventil gebildet sein, dessen einer Ventilsitz durch den Außenumfang des Innenkörpers 41 und dessen anderer, demgegenüber bewegbarer Ventilsitz durch den Innenumfang einer Manschette des Kolbens gebildet ist, welche von dem Innenkörper durchsetzt ist. Die Ventilsitze können stattdessen oder zusätzlich auch durch Stirnflächen des Innenkörpers und des demgegenüber gegen die Kraft einer Ventilfeder verschiebbaren Kolbens gebildet sein und beispielsweise am vorderen bzw. inneren Ende des Kolbens liegen. Desweiteren kann der Körper 5 bzw. der Speicher 22 in der Ausgangsstellung oder am Ende des Pumphubes bis in den Stutzen 6 hineinragen. Zwischen den Körpern 4, 5 bzw. 4, 22 bzw. 4, 26 kann eine form- und/oder kraftschlüssig wirkende Sicherung gegen Abziehen bzw. Auseinanderziehen vorgesehen sein, welche gegebenenfalls, z.B. bajonettverschlußartig durch eine Drehbewegung, entriegelbar ist. Statt des jeweiligen Mantels können auch nur über den Umfang verteilte, im Abstand zueinanderliegende Mantelabschnitte vorgesehen sein, die bei entsprechend großem Zwischenabstand nur frei vorstehende Arme bilden. Durch die beschriebene Form der beiden Körper 4, 5 bzw. der Mäntel 33, 19 sind diese gegeneinander verdrehgesichert.

Die Öffnungen 7 sind mit einem kappenförmigen Verschluß 56 verschließbar, dessen offene Endfläche in Schließlage an der Außenseite der Stirnwand 34 zwischen den Handhaben 8 anliegt und der über eine Schnappverbindung kraftabhängig gesichert in den Außenumfang des Fußes des Stutzens 6 eingreift. Der Innenumfang des Mantels des Verschlusses 56 ist über dessen Länge durchgehend kreisrund bzw. gleichförmig polygonal, wobei der Verschluß 56 den gesamten Stutzen 6 zumindest dicht gegen Staub abschirmt. Die beiden Fördereinheiten 15 können gleich oder unterschiedlich ausgebildet sein.

Alle genannten Eigenschaften und dergleichen können genau wie beschrieben, nur annähernd bzw. im wesentlichen wie beschrieben oder auch stärker davon abweichend vorgesehen sein. Ferner kann jede Anordnung und jeder Bauteil nur in einfacher Anordnung oder zwei- bzw. mehrfach vorgesehen sein.

## Patentansprüche

1. Austrag-Einheit für Medien, insbesondere zur intranasalen Behandlung, mit
• mindestens einem Austragförderer (23),
• einer Austragsbetätigung mit mehreren Betätigungsstellungen,
• wenigstens einem Auslaßkanal (37),
• zwei Auslaßöffnungen (7) für das Medium und
• mindestens einer in wenigstens einen Grundkörper (4,5) eingesetzten Baueinheit (35), wobei
• an dem Grundkörper eine Stirnwand (34) ausgebildet ist, **dadurch gekennzeichnet, dass**
• mindestens eine Auslaßöffung (7) durch einen Ausgang einer Zerstäuberdüse (48) gebildet bzw. als Austragsöffnung (7) ausgebildet ist,
• der Grundkörper (4) einen Stutzen (6) aufweist, an dessen freiem Ende die Auslaßöffnungen (7) ausgebildet sind,
• die Baueinheit (35) einen Innenkörper (41), der sich annähernd bis zum freien Ende des Stutzens (6) erstreckt, und ein Tragteil (45) aufweist, wobei zwischen Stutzen (6) und Innenkörper (41) Kanalabschnitte (40) des wenigstens einen Auslaßkanals (37) ausgebildet sind,
und die Baueinheit (35) nur über den Tragteil (45) und zwar mit einer Befestigung (46) an der Innenseite der Stirnwand (34), gehaltert ist.

2. Einheit nach Anspruch 1, **dadurch gekennzeichnet, daß** mindestens eine Auslaßöffnung (7) ins Freie führend das Medium nach außen von der Einheit (1) freigibt, daß insbesondere mindestens zwei Austragöffnungen (7) unmittelbar nebeneinanderliegend gleichgerichtet sind, und daß vorzugsweise wenigstens zwei Austragöffnungen (7) zur Medienmischung im Freien oder dgl. Ausgänge gesonderter Zerstäuberdüsen (48), Wirbeleinrichtungen (50), Innenkörper (41) bzw. Auslaßkanäle (37) bilden.

3. Einheit nach Anspruch 1 oder 2, **dadurch gekennzeichnet, daß** mindestens zwei gesonderte Auslaßkanäle (37) von mindestens einem eine Baueinheit bildenden und in wenigstens eine Aufnahmeöffnung (42) eingesetzten Innenkörper (41) begrenzt sind, daß insbesondere mindestens zwei Innenkörper (41) der Baueinheit (35) in gesonderten Aufnahmeöffnungen (42) liegen, und daß vorzugsweise zwei Innenkörper (41) nur im Bereich eines Endes (44) zur Bildung der Baueinheit (35) miteinander verbunden sind.

4. Einheit nach einem der vorhergehenden Ansprüche, **dadurch gekennzeichnet, daß** mindestens ein, wenigstens einen Auslaßkanal (37) begrenzender, Innenkörper (41) im Bereich des stromaufwärtsliegenden Endes (44) in einen über seinen Außenumfang vorstehenden Sicherungsteil (45) übergeht, daß insbesondere zwei gesonderte, achsparallel nebeneinander liegende Innenkörper (41) einteilig mit einem gemeinsamen Verbindungsteil (45) ausgebildet sind, und daß vorzugsweise der Sicherungs- bzw. Verbindungsteil (45) an einem Grundkörper (4) festsitzend angeordnet ist.

5. Einheit nach einem der vorhergehenden Ansprüche, **dadurch gekennzeichnet, daß** ein Grundkörper (4) als wenigstens einen Innenkörper (41) einschließende Baueinheit einen Anschluß (36) für mindestens einen als vormontierte Fördereinheit (15, 22), wie eine Pumpe, ein Pumpenzylinder oder dergleichen, ausgebildeten Austragförderer (23) trägt, daß insbesondere der Anschluß (36) ein Betätigungselement (30) des Austragförderers (23) bildet, und daß vorzugsweise der Anschluß (36) einen an einen Austragkanal (31) einer Druckkammer (25) des Austragförderers (23) anzuschließenden Abschnitt (38) eines Auslaßkanales (37) bildet und/oder daß mindestens ein Anschluß (36) für eine Fördereinheit (15) eine Anschlußöffnung für den festsitzenden Eingriff eines Betätigungselementes (30) eines Austragförderers (23) aufweist, daß insbesondere der Anschluß (36) hülsenförmig ist, und daß vorzugsweise der Anschluß (36) gegenüber einem Innenkörper (41) seitlich versetzt ist.

6. Einheit nach einem der vorhergehenden Ansprüche, **dadurch gekennzeichnet, daß** ein an einem Grundkörper (4) als gesonderte Baueinheit anzuordnender Tragteil (45) an einer Seite vorstehend mindestens einen Innenkörper (41) und an der davon abgekehrten anderen Seite vorstehend mindestens einen Anschluß (36) für eine Fördereinheit (15) oder dergleichen aufweist, daß insbesondere der Tragteil (45) plattenförmig ausgebildet quer zur Mittelachse des Innenkörpers (41) bzw. des Anschlußes (36) liegt, und daß vorzugsweise der Tragteil (45) vollständig innerhalb des Grundkörpers (4) liegend unmittelbar an einer plattenförmigen Wand (34) oder dergleichen befestigt ist.

7. Einheit nach einem der vorhergehenden Ansprüche, **dadurch gekennzeichnet, daß** mindestens eine Baueinheit (35) quer aneinander anschließende Kanalabschnitte (38, 39, 40) wenigstens eines Auslaßkanales (37) begrenzt, daß insbesondere zwei etwa parallele Längsabschnitte (38, 40) über einen Querkanal (39) aneinander anschließen, und daß vorzugsweise der Querkanal (39) im Querschnitt von einem Grundkörper (4) und der Baueinheit (35) begrenzt ist.

8. Einheit nach einem der vorhergehenden Ansprüche, **dadurch gekennzeichnet, daß** ein erster von zwei Grundkörpern (4, 5) beiderseits eines frei vorstehenden, von mindestens einer Austragöffnung (7) durchsetzten Austragstutzens (6) stirnseitig Handhaben (8) und entgegengesetzt zum Austragstutzen (6) von den Handhaben (8) abstehend einen Kappenmantel (33) einer eine Stirnwand (34) aufweisenden Betätigungskappe bildet, innerhalb welcher die Baueinheit (35) an eine Stirnwand (34) anschließend befestigt ist, daß insbesondere die Baueinheit (35) in den Austragstutzen (6) über die Ebene der Handhaben (8) hinausreichend eingreift, und daß vorzugsweise die Baueinheit (35) zur im wesentlichen einzigen Betätigungs-Verbindung des Grundkörpers (4) mit mindestens einem Austragförderer (23) ausgebildet ist.

9. Einheit nach einem der vorhergehenden Ansprüche, **dadurch gekennzeichnet, daß** ein zweiter von zwei Grundkörpern (4, 5) mindestens einen Medienspeicher (22) enthält, daß insbesondere der Medienspeicher (22) wenigstens mit gegenüber ihm mit der Austrag-Betätigung bewegbaren Teilen (26) mindestens eines Austragförderers (23) eine vormontierte Montageeinheit zur Verbindung mit dem jeweiligen Grundkörper (4, 5) bildet, und daß vorzugsweise der zweite Grundkörper (5) eine Speicher-Aufnahme (18) für mindestens einen gesonderten Medien-Speicher (22) aufweist.

10. Einheit nach einem der vorhergehenden Ansprüche, **dadurch gekennzeichnet, daß** ein zweiter Grundkörper (5) zwei Speicheraufnahmen (18) für gesonderte, achsparallel nebeneinander liegende Montage- bzw. Fördereinheiten (15) und ein erster Grundkörper (4) gesonderte Anschlüsse (36) sowie Auslaßkanäle (37) für zwei Austragförderer (23) aufweist, daß insbesondere die beiden Grundkörper (4, 5) einander übergreifen bzw. in Axialansicht langgestreckt sind, und daß vorzugsweise äußerste Gehäusemäntel (33, 19) des ersten und des zweiten Grundkörpers (4, 5) zur gegenseitigen Drehsicherung od. dgl. einander in wenigstens einer Betätigungsstellung mit Umfangsflächen übergreifen.

11. Einheit nach einem der vorhergehenden Ansprüche, **dadurch gekennzeichnet, daß** mindestens eine Auslaßöffnung (7) eine in Axialansicht langgestreckte Stirnwand (47) durchsetzt, daß insbesondere die Stirnwand (47) durch das freie Ende eines langgestreckten Austragstutzens (6) gebildet ist, und daß vorzugsweise der Außenquerschnitt des Austragstutzens (6) von einer annähernd ovalen Form der Stirnwand (47) zum stromaufwärtsliegenden Ende im wesentlichen stetig in eine gleichmäßiger gekrümmte bis kreisrunde Form übergeht.

## Claims

1. A discharge unit for media, particularly for intranasal treatment having
• at least one discharge delivery means (23),
• a discharge actuator including several actuating positions,
• at least one discharge passage (37),
• two discharge openings (7) for the medium and
• at least one module (35) inserted in at least one base body (4, 5), a face end wall (34) being configured on said base body (4, 5)
**characterized in that**
• at least one discharge opening (7) is formed by an output of an atomizer nozzle (48) or is configured as a discharge opening (7),
• said base body (4) comprises a port (6) at the free end of which said discharge openings (7) are configured,
• said module (35) comprises an internal body (41) extending almost up to the free end of said port (6), and a carrier part (45), passage sections (40) of said at least one discharge passage (37) being configured between said port (6) and said internal body (41),
and said module (35) is supported only by said carrier part (45), i.e. by a fastener (46) at the inner side of said face end wall (34).

2. The unit as set forth in claim 1, **characterized in that** at least one discharge orifice (7) leading to atmosphere liberates the medium outwardly from said unit (1), in particular at least two discharge orifices (7) actuating in the same direction directly juxtaposed and preferably at least two discharge orifices (7) for blending the media in the atmosphere or the like forming outputs of separate atomizer nozzles (48), swirlers (50), internal bodies (41) and/or discharge passage (37).

3. The unit as set forth in claim 1 or 2, **characterized in that** at least two separate outlet passages (37) are defined by at least one internal body (41) forming one module and inserted in at least one supporting opening (42) are defined, in particular at least two internal bodies (41) of a module (35) being located in separate supporting opening (42) and preferably two internal bodies (41) being connected to each other only in the region of one end (44) for forming a module (35).

4. The unit as set forth in any of the preceding claims, **characterized in that** at least one internal body (41) defining at least one outlet passage (37) in the region of the upstream end (44) translates into a locking part (45) protruding past its outer circumference, in particular two separate internal bodies (41) located axially parallel juxtaposed being configured integrally with a common connecting part (45) and preferably said locking or connecting part (45) being disposed firmly seated on a base body (4).

5. The unit as set forth in any of the preceding claims, **characterized in that** a base body (4) as a module encompassing at least one internal body (41) carries a connector (36) for at least one discharge delivery means (23) configured as a pre-assembled module (15, 22), such as a pump, a pump barrel or the like, in particular said connector (36) forming an actuating element (30) of said discharge delivery means (23) and preferably said connector (36) forming a section (38) of an outlet passage (37) to be connected to a discharge passage (31) of a pressure chamber (25) of said discharge delivery means (23) and/or at least one connection (36) for a delivery unit (15) comprises a connecting opening for the firmly-seated engagement of an actuating element (30) of a discharge delivery means (23), in particular one connection (36) being sleeve-shaped and preferably one connection (36) being offset to one side with respect to an internal body (41).

6. The unit as set forth in any of the preceding claims, **characterized in that** a carrier part (45) to be arranged on a base body (4) as a separate module comprises protruding from one side at least one internal body (41) and protruding from the other side facing away from the latter a connection (36) for a delivery unit (15) or the like, in particular said carrier part (45) being located configured plate-shaped transverse to the center line of said internal body (41) and/or of said connection (36), and preferably said carrier part (45) being secured directly to a plate-shaped wall (34) or the like completely within said base body (4).

7. The unit as set forth in any of the preceding claims, **characterized in that** at least one module (35) defines transversely juxtaposed passage sections (38, 39, 40) of at least one outlet passage (37), in particular two approximately parallel longitudinal sections (38, 40) adjoining each other via a transverse passage (39) and preferably said transverse passage (39) being limited in cross-section by a base body (4) and a module (35).

8. The unit as set forth in any of the preceding claims, **characterized in that** a first of two base bodies (4, 5) on both sides of a freely projecting discharge port (6) passed through by at least one discharge orifice (7) forms face end grips (8) and opposing said discharge port (6), protruding from said grips (8) a cap shell of an actuating cap comprising a face end wall (34), within which a module (35) is secured adjoining a face end wall (34), in particular said module (35) engaging in said discharge port (6) extending past the plane of said grips (8) and preferably said module (35) being configured with at least one discharge delivery means (23) for the substantially sole actuating connection of said base body (4).

9. The unit as set forth in any of the preceding claims, **characterized in that** a second of two base bodies (4, 5) contains at least one media reservoir (22), in particular the media reservoir (22) forms with at least parts (26) movable with respect thereto by the discharge actuation of at least one discharge delivery means (23) a pre-assembled fitting unit for connecting to the base body (4, 5), and preferably said second base body (5) comprising a reservoir support (18) for at least one separate media reservoir (22).

10. The unit as set forth in any of the preceding claims, **characterized in that** a second base body (5) comprises two reservoir supports (18) for separate assembly or delivery units (15) located juxtaposed axially in parallel and a first base body (4) comprises separate connections (36) as well as outlet passages (37) for two discharge delivery means (23), in particular said two base bodies (4, 5) clasping each other and/or being elongated axially and preferably the outermost housing shells (33, 19) of said first and said second base body (4, 5) clasping each other in at least one actuating position by circumferential surfaces for mutual rotational securing or the like.

11. The unit as set forth in any of the preceding claims, **characterized in that** at least one discharge orifice (7) passes through a face end wall (47) elongated axially, in particular said face end wall (47) being formed by the free end of an elongated discharge port (6) and preferably the outer cross-section of said discharge port (6) translating substantially continuously into a consistently curved up to a circular shape from an oval shape of said face end wall (47) up to the upstream end.

## Revendications

1. Unité de décharge pour matières, notamment pour un traitement intranasal, avec
■ au moins un transporteur de décharge (23),
■ un actionnement de décharge avec plusieurs positions d'actionnement,
■ au moins un conduit d'échappement (37),
■ deux ouvertures d'échappement (7) pour la matière et
■ au moins une unité constructive (35) insérée dans au moins un corps de base (4, 5), une paroi frontale (34) étant réalisée sur le corps de base,
**caractérisée en ce que**
• au moins une ouverture d'échappement (7) est formée par une sortie d'une buse de pulvérisation (48) ou encore réalisée en forme d'ouverture de décharge (7),
• le corps de base (4) présente une tubulure (6), à l'extrémité libre de laquelle sont réalisées les ouvertures d'échappement (7),
• l'unité constructive (35) présente un corps intérieur (41), qui s'étend à peu près jusqu'à l'extrémité libre de la tubulure (6), et une pièce de support (45), des sections de conduit (40) du conduit d'échappement (37) au moins unique étant réalisées entre la tubulure (6) et le corps intérieur (41),et l'unité constructive (35) étant soutenue uniquement par la pièce de support (45) et plus précisément avec un moyen de fixation (46) à la face intérieure de la paroi frontale (34).

2. Unité d'après la revendication 1, **caractérisée en ce qu'**au moins une ouverture d'échappement (7) relâche la matière vers l'extérieur à partir de l'unité (1) en la conduisant à l'extérieur, **en ce que** notamment au moins deux ouvertures de décharge (7) sont orientées dans la même direction en étant situées directement l'une à côté de l'autre, et **en ce que** de préférence au moins deux ouvertures de décharge (7) forment, pour le mélange des matières à l'air libre ou similaires, des sorties de buses de pulvérisation (48) individuelles, de moyens de tourbillonnement (50), de corps intérieurs (41) ou encore de conduits d'échappement (37).

3. Unité d'après la revendication 1 ou 2, **caractérisée en ce qu'**au moins deux conduits d'échappement (37) individuels sont délimités par au moins un corps intérieur (41) constituant une unité constructive et étant inséré dans au moins une ouverture de logement (42), **en ce que** notamment au moins deux corps intérieurs (41) de l'unité constructive (35) se trouvent dans des ouvertures de logement (42) individuelles, et **en ce que** de préférence deux corps intérieurs (41) sont raccordés entre eux uniquement dans le domaine d'une extrémité (44) pour la formation de l'unité constructive (35).

4. Unité d'après une des revendications précédentes, **caractérisée en ce qu'**au moins un corps intérieur (41) délimitant au moins un conduit d'échappement (37) se transforme, dans le domaine de l'extrémité (44) située en amont, en une pièce d'assurage (45) saillant au-delà de son périmètre extérieur, **en ce que** notamment deux corps intérieurs (41) individuels et situés l'un à côté de l'autre de manière parallèle à l'axe, sont réalisés d'une seule pièce avec une pièce de raccordement (45) commune, et **en ce que** de préférence la pièce d'assurage ou encore de raccordement (45) est disposée de manière fixe sur un corps de base (4).

5. Unité d'après une des revendications précédentes, **caractérisée en ce qu'**un corps de base (4) porte, en tant qu'unité constructive comprenant au moins un corps intérieur (41) une jonction (36) pour au moins un transporteur de décharge (23), réalisé en tant qu'unité de transport (15, 22) prémontée, telle qu'une pompe, un cylindre de pompe ou similaires, **en ce que** notamment la jonction (36) forme un élément d'actionnement (30) du transporteur de décharge (23), et **en ce que** de préférence la jonction (36) forme une section (38) d'un conduit d'évacuation (37), laquelle est à raccorder au conduit de décharge (31) d'une chambre sous pression (25) du transporteur de décharge (23), et/ou **en ce qu'**au moins une jonction (36) pour une unité de transport (15) présente une ouverture de raccordement pour l'engagement indesserrable d'un élément d'actionnement (30) d'un transporteur de décharge (23), **en ce que** notamment la jonction (36) est en forme de douille, et **en ce que** de préférence la jonction (36) est décalée latéralement par rapport au corps intérieur (41).

6. Unité d'après une des revendications précédentes, **caractérisée en ce que** une pièce de support (45), qui est à disposer sur un corps de base (4) en tant qu'unité constructive individuelle, présente de manière saillante sur un côté au moins un corps intérieur (41) et de manière saillante du côté opposé au moins une jonction (36) pour une unité de transport (15) ou similaires, **en ce que** notamment la pièce de support (45) se trouve, en étant réalisée en forme de plaque, transversalement par rapport à l'axe médian du corps intérieur (41) ou encore de la jonction (36), et **en ce que** de préférence la pièce de support (45) est fixée, en étant entièrement située à l'intérieur du corps de base (4), directement auprès d'une parois (34), ou similaires, en forme de plaque.

7. Unité d'après une des revendications précédentes, **caractérisée en ce qu'**au moins une unité constructive (35) délimite des sections de conduit (38, 39, 40) transversalement adjacentes l'une à l'autre, d'au moins un conduit d'échappement (37), **en ce que** notamment deux sections longitudinales (38, 40) à peu près parallèles sont reliées l'une à l'autre au moyen d'un conduit transversal (39), et **en ce que** le conduit transversal (39) est délimité en section transversale par un corps de base (4) et par l'unité constructive (35).

8. Unité d'après une des revendications précédentes, **caractérisée en ce que** le premier de deux corps de base (4, 5) forme, sur les deux côtés d'une tubulure de décharge (6) librement saillante et traversée par au moins une ouverture de décharge (7), des moyens de manipulation (8) sur le côté frontal et une enveloppe de capuchon (33), située au côté opposé à la tubulure de décharge (6) et à l'écart des moyens de manipulation (8), d'un capuchon d'actionnement présentant une paroi frontale (34), à l'intérieur de laquelle enveloppe est fixée l'unité constructive (35) faisant suite à une paroi frontale (34), **en ce que** notamment l'unité constructive (35) s'engage dans la tubulure de décharge (6) en s'étendant au-delà du plan des moyens de manipulation (8), et **en ce que** de préférence l'unité constructive (35) est réalisée pour le raccordement d'actionnement essentiellement unique du corps de base (4) avec au moins un transporteur de décharge (23).

9. Unité d'après une des revendications précédentes, **caractérisée en ce que** le deuxième des deux corps de base (4, 5) contient au moins un réservoir à matières (22), **en ce que** notamment le réservoir à matières (22) forme, au moins avec des parties (26) d'au moins un transporteur de décharge (23), lesquelles peuvent être déplacées avec le moyen d'actionnement de décharge par rapport au réservoir, une unité de montage prémontée pour le raccordement avec le respectif corps de base (4, 5), et **en ce que** de préférence le deuxième corps de base (5) présente un logement de réservoir (18) pour au moins un réservoir à matières (22) individuel.

10. Unité d'après une des revendications précédentes, **caractérisée en ce qu'**un deuxième corps de base (5) présente deux logements de réservoir (18) pour des unités de montage ou encore de transport (15) individuelles et situées l'une à côté de l'autre parallèlement à l'axe, et qu'un premier corps de base (4) présente des jonctions (36) individuelles ainsi que des conduits d'échappement (37) pour deux transporteurs de décharge (23), **en ce que** notamment les deux corps de base (4, 5) s'engagent l'un au-dessus de l'autre ou encore qu'ils sont de forme allongée en vue axiale, et **en ce que** de préférence les enveloppes de boîtier (33, 19) les plus extérieures du premier et du deuxième corps de base (4, 5) s'engagent l'une au-dessus de l'autre par les surfaces circonférencielles, pour un assurage rotationnel réciproque ou similaires, au moins dans une position d'actionnement.

11. Unité d'après une des revendications précédentes, **caractérisée en ce qu'**au moins une ouverture d'échappement (7) traverse une paroi frontale (47) de forme allongée en vue axiale, **en ce que** notamment la paroi frontale (47) est constituée par l'extrémité libre d'une tubulure de décharge (6) de forme allongée, et **en ce que** de préférence la section transversale extérieure de la tubulure de décharge (6) se convertit, à partir d'une forme approximativement ovale de la paroi frontale (47), en direction de l'extrémité située en amont, de manière essentiellement continue en une forme uniformément courbée jusqu'à circulaire.
